(19) Europäisches Patentamt
European Patent Office
Office européen des brevets

(11) Veröffentlichungsnummer: **0 244 705**
**A2**

(12) # EUROPÄISCHE PATENTANMELDUNG

(21) Anmeldenummer: **87105981.2**

(22) Anmeldetag: **24.04.87**

(51) Int. Cl.[4]: **C 07 D 277/82**, C 07 D 327/04, A 01 N 43/74

(30) Priorität: **30.04.86 DE 3614595**

(43) Veröffentlichungstag der Anmeldung: **11.11.87**
**Patentblatt 87/46**

(84) Benannte Vertragsstaaten: **AT BE CH DE ES FR GB GR IT LI LU NL SE**

(71) Anmelder: **HOECHST AKTIENGESELLSCHAFT, Postfach 80 03 20, D-6230 Frankfurt am Main 80 (DE)**

(72) Erfinder: **Heubach, Günther, Dr., Luisenstrasse 15, D-6233 Kelkheim (Taunus) (DE)**
Erfinder: **Matterstock, Karl, Dr., Lessingstrasse 38, D-6238 Hofheim am Taunus (DE)**
Erfinder: **Mildenbergerg, Hilmar, Dr., Fasanenstrasse 24, D-6233 Kelkheim (Taunus) (DE)**
Erfinder: **Sachse, Burkhard, Dr., An der Ziegelei 30, D-6233 Kelkheim (Taunus) (DE)**

(54) **Benzoxazolyl- und Benzthiazolylaniline, Verfahren zu ihrer Herstellung und ihre Verwendung als fungizide Mittel.**

(57) Die Verbindungen der Formel I,

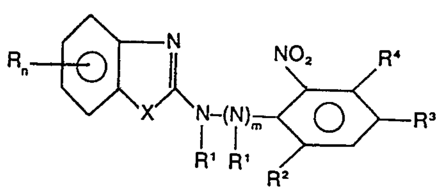

I

worin

X = O oder S, R = Halogen, CN, SCN, $NO_2$, $(C_1-C_4)$-Alkyl, $(C_1-C_4)$Alkoxy, $(C_1-C_4)$Alkylthio, die haolgeniert sein können, oder (substituiertes) Phenoxy,

$R^1$ = H, Alkyl oder Alkyl-carbonyl $R^2$, $R^3$ = Halogen, $NO_2$, Haloalkyl, CN, COOH oder Alkoxy-carbonyl,

$R^4$ = H, Halogen, (halogeniertes) Alkyl,

m = 0 oder 1 und

n = 0 bis 4

bedeuten, oder deren Salze, mit der Maßgabe, daß nicht beide Reste $R^2$, $R^3$ gleichzeitig im selben Molekül $NO_2$ bedeuten dürfen, besitzen vorteilhafte fungizide Wirkungen.

EP 0 244 705 A2

# Benzoxazolyl- und Benzthiazolylaniline, Verfahren zu ihrer Herstellung und ihre Verwendung als fungizide Mittel

Es ist bekannt, daß gewisse Pyridylaniline und Pyrimidinylaniline, wie sie in EP-A 31 257 und in GB-A 2 137 991 beschrieben sind, microbicide Eigenschaften aufweisen. Es wurden nun neue Benzoxazolyl- und Benzthiazolylanilin-Derivate gefunden, die vorteilhafte fungizide Wirkungen besitzen.

Gegenstand der Erfindung sind daher die Verbindungen der Formel I,

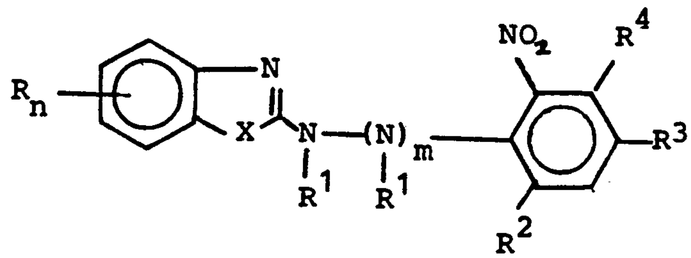

I

worin X = O oder S,

R = Halogen, CN, SCN, $NO_2$, $(C_1-C_4)$Alkyl, $(C_1-C_4)$Alkoxy, $(C_1-C_4)$Alkylthio, wobei die drei letztgenannten Reste ganz oder teilweise halogeniert sein können, oder Phenoxy, das durch Halogen, $(C_1-C_4)$Alkyl, $(C_1-C_4)$Alkoxy, Halo$(C_1-C_4)$alkoxy oder Halo$(C_1-C_4)$alkyl substituiert sein kann,

$R^1$ = jeweils unabhängig voneinander H, $(C_1-C_4)$Alkyl oder $(C_1-C_4)$Alkyl-carbonyl

$R^2$, $R^3$ = jeweils unabhängig voneinander Halogen, $NO_2$, Halo$(C_1-C_4)$alkyl, CN, COOH oder $(C_1-C_4)$-Alkoxy-carbonyl,

$R^4$ = H, Halogen, $(C_1-C_4)$Alkyl oder Halo$(C_1-C_4)$alkyl

m = 0 oder 1 und

n = 0 bis 4

bedeuten, oder deren Salze, mit der Maßgabe, daß nicht beide Reste $R^2$, $R^3$ gleichzeitig im selben Molekül $NO_2$ bedeuten dürfen.

Bevorzugt unter den Verbindungen der Formel I sind solche, bei denen R in Position 4 oder 6 des Benzoxazolyl- bzw. Benzthiazolylrestes steht und F, Cl oder $CF_3$ bedeutet und worin $R^2$= $NO_2$ oder $CF_3$, $R^3$ = $CF_3$, $CCl_3$, CN, COOH, $(C_1-C_4)$-Alkoxycarbonyl, Nitro, $R^4$ = H oder Halogen, m = 0 und n = 1 oder 2 bedeuten.
Besonders bevorzugt sind die Verbindungen der Formel I mit $(R_n)$ = 6-Cl oder 6-$CF_3$; $R^2$ = $NO_2$ und $R^3$ = $CF_3$, $R^4$ = Cl.

Als halogenierte Alkylreste kommen beispielsweise in Betracht $CF_3$; $CHF_2$; $CH_2F$; $CCl_3$; $CHCl_2$; $CH_2Cl$; $CBr_3$; $CF_2Cl$; $CClF_2$; $CF_2CHF_2$; $CF_2CF_3$; $CF_2CHClF$; $CF_2CHCl_2$; $CCl_2CCl_3$; n-$C_3F_7$; $CH(CF_3)_2$; n-$C_4F_9$,sec. $C_4F_9$.
Die Salzbildung ist insbesondere im Falle $R^2$ oder $R^3$ = COOH möglich. Es werden alle in der Landwirtschaft einsetzbaren Salze von der Erfindung umfaßt, beispielsweise Alkali-, Erdalkali-Salue (Na-, K-, Mg-, Ca-Salz), Ammonium- oder substituierte Ammoniumsalze.
Halogen, "halogeniert" oder "Halo" bedeutet die Substituenten F, Cl, Br oder J, bevorzugt F oder Cl.
Gegenstand der Erfindung sind ferner Verfahren zur Herstellung der Verbindungen der Formel I, dadurch gekennzeichnet, daß man

a) eine Verbindung der Formel II

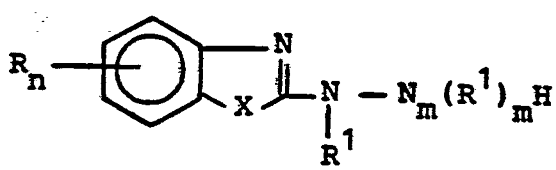

(II)

mit einer Verbindung der Formel III,

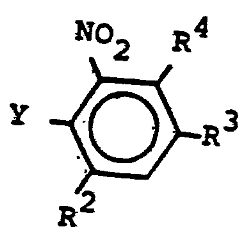

(III)

worin Y Halogen bedeutet oder

b) eine Verbindung der Formel IV

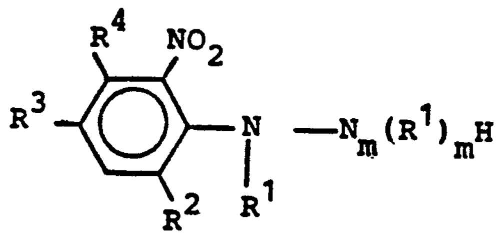

(IV)

mit einer Verbindung der Formel V,

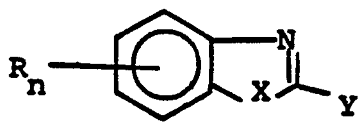

(V)

worin Y die Bedeutung wie in Formel III besitzt, umsetzt.

Vorzugsweise werden die Verbindungen in Gegenwart einer Base und eines oder mehrerer Lösungs- oder Verdünnungsmittel hergestellt.
Geeignete inerte Lösungsmittel sind aliphatische oder aromatische Kohlenwasserstoffe wie Benzol, Toluol, Xylol und Petrolether. Halogenierte Kohlenwasserstoffe wie Chlorbenzol, Methylenchlorid, Ethylenchlorid, Chloroform, Tetrachlorkohlenstoff oder Tetrachlorethylen, Ether wie Dialkylether, Anisol, Tetrahydrofuran und Diethylenglykoldimethylether; Nitrile wie Acetonitril, Propionitril, N,N-dialkylierte Amide wie Dimethylformamid, Dimethylsulfoxid und Ketone wie z.B. Aceton oder Methylethylketon.

Geeignete Basen sind die dem Fachmann geläufigen Verbindungen wie (Erd)alkalimetallcarbonate wie Kaliumcarbonat, Ca-carbonat oder (Erd)alkalimetallhydroxide wie Kaliumhydroxid, Natriumhydroxid, Calciumhydroxid.

Die Temperatur bei der Umsetzung hängt von der Auswahl der Reaktionspartner, des Lösungs- oder Verdünnungsmittels und der Base ab.
Im allgemeinen liegt die Reaktionstemperatur im Intervall von -30°C bis 130°C, bevorzugt bei Temperaturen von -20°C bis 80°C.

Die Verbindungen II und III bzw. IV und V werden in etwa stöchiometrischen Mengen eingesetzt, wobei eine Komponente auch in einem Überschuß bis zu 10 % angewandt werden kann.

Das säurebindende Mittel wird in mindestens stöchiometrischer Menge, meist jedoch im Überschuß eingesetzt.
Die Aufarbeitung erfolgt in üblicher Weise bei mit Wasser nicht mischbaren Lösungsmitteln durch Ausschütteln mit Wasser, Trocknen der organischen Phase und Eindampfen. Bei mit Waser mischbaren Lösungsmitteln durch Verdünnen mit Wasser und Extrahieren des Produkts mit einem nicht wassermischbaren Lösungsmittel.

Die Verbindungen der Formel I fallen kristallin oder als nicht destillierbare Öle an. Zur weiteren Reinigung kristallisiert man aus einem geeigneten Lösungsmittel um oder man reinigt die Produkte durch Chromatographie über Kieselgel.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich wichtiger, phytopathogener Pilze, wie z.B. Piricularia oryzae, Echte Mehltauarten, Fusariumarten, Plasmopora viticola, Pseudoperonospora cubensis, verschiedene Rostpilze und Pseudocercosporella herpotrichoides. Besonders gut werden Benzimidazol- und Dicarboximid-sensible und -resistente Botrytis cinerea-Stämme erfaßt.

Die Verbindungen der Formel I eignen sich auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder alkylphenylsulfonate und Dispergiermittel, z.B. ligninsulfonsaures Natrium, 2,2'-dinaphthylmethan-6,6'disulfonsaures Natrium, dibutylnaphthalinsulfonsaures Natrium oder auch oleoylmethyltaurinsaures Natrium enthalten. Ihre Herstellung erfolgt in üblicher Weise, z.B. durch Mahlen und Vermischen der Komponenten. Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:
Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitan-fettsäureester oder Polyoxethylen-sorbitester.

Stäubemittel kann man durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemittel, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen.
Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe us. verwendet werden.

Daneben enthalten die genannten Wirkstoffformulierungen gegebenenfalls die jeweils üblichen Haft-, Netz-, Dispergier-, Emulgier-, Penetrations-, Lösungsmittel-, Füll- oder Trägerstoffe.

Die Anwendungsdosierungen können innerhalb weiter Grenzen je nach Klima, Bodenverhältnissen, Art des zu verwendeten Präparates schwanken. Sie liegen im allgemeinen zwischen 1000 und 100 g/ha, insbesondere zwischen 500 und 125 g Wirkstoff/ha.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich. Insbesondere bei Mischungen mit Fungiziden werden teilweise auch synergetische Wirkungssteigerungen erzielt.

Als Kombinationspartner eignen sich die folgenden Verbindungen:

Imazalil, Prochloraz, Fenapanil, SSF105, Triflumizol, PP969, Flutriafol, BAY-MEB 6401,
Propiconazol, Etaconazol, Diclobutrazol, Bitertanol, Triadimefon, Triadimenol, Fluotrimazol, Tridemorph, Dodemorph, Fenpropimorph, Falimorph, S-32165,
Chlobenzthiazone, Parinol, Buthiobat, Fenpropidin, Triforine, Fenarimol,
Nuarimol, Triarimol, Ethirimol, Dimethirimol,

Bupirimate, Rabenzazole, Tricyclazole, Ofurace, Furalaxyl, Benalaxyl, Metalaxyl, Pencyuron, Oxadixyl, Cyprofuram, Dichlomezin, Probenazole, Fluobenzimine, Pyroxyfur, NK-483, PP-389, Pyroquilon, Hymexazole, Fenitropan, UHF-8227, Tolclofosmethyl, Ditalimfos, Edifenphos, Pyrazophos, Isoprothiolane, Cymoxanil, Dichloruanid, Captafol, Captan, Folpet, Tolylfluanid, Chlorothalonil, Etridiazol, Iprodione, Procymidon, Vinclozolin, Metomeclan, Myclozolin, Dichlozolinate, Fluorimide, Drazoxolon, Chinomethionate, Nitrothalisopropyl, Dithianon, Dinocap, Binapacryl, Fentinacetate, Fentinhydroxide, Carboxin, Oxycarboxin, Pyracarbolid, Methfuroxam, Fenfuram, Furmecyclox, Benodanil, Mebenil, Mepronil, Flutolanil, Fuberidazole, Thiabendazole, Carbendazim, Benomyl, Thiofanate, Thiofanate-methyl, CGD-94240F, IKF-1216, Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cu-oxychlorid, Oxine-Cu, Cu-oxide, Schwefel, Fosetylaluminium,

Natrium-dodecylbenzolsulfonat,
Natrium-dodecylsulfat,,
Natrium-C13/C15-alkoholethersulfonat,
Natrium-cetostearylphosphatester,
Dioctyl-natriumsulfosuccinat,
Natrium-isopropylnaphthalinsulfonat,
Natrium-methylenbisnaphthalinsulfonat,
Cetyl-trimethyl-ammoniumchlorid,
Salze von langkettigen primären, sekundären oder tertiären Aminen,
Alkyl-propylenamine,
Lauryl-pyridiniumbromid,
Ethoxilierte quaternierte Fettamine,,
Alkyl-dimethyl-benzyl-ammoniumchlorid und

1-Hydroxyethyl-2-alkyl-imidazolin. Bezüglich der Nummernpräparate s. Deutsche Patentanmeldung P 36 09 645.8.

Die Erfindung wird durch die folgenden Beispiele erläutert.

A. Formulierungsbeispiele

Beispiel A:

Ein Stäubemittel wird erhalten, indem man
10 Gewichtsteile Wirkstoff
und 90 Gewichtsteile Talkum als Inertstoff
mischt und in einer Schlagmühle zerkleinert.

Beispiel B:

Ein in Wasser leicht dispergierbares, benetzbares Pulver wird erhalten, indem man

25 Gewichtsteile Wirkstoff
64 Gewichtsteile kaolinhaltigen Quarz als Inerstoff
10 Gewichtsteile ligninsulfonsaures Kalium
und 1 Gewichtsteil oleoylmethyltaurinsaures Natrium als Netz- und Dispergiermittel
mischt und in einer Stiftmühle mahlt.

Beispiel C:

Ein in Wasser leicht dispergierbares Dispersionskonzentrat wird erhalten, indem man
20 Gewichtsteile Wirkstoff mit
6 Gewichtsteilen Alkylphenolpolyglykoläther (®Triton X 207)
3 Gewichtsteilen Isotridencanolpolyglykoläther (8 AeO)

und 71 Gewichtsteilen paraffinischem Mineralöl (Siedebereich z.B. ca. 255 bis über 377°C) mischt und in einer Reibkugelmühle auf eine Feinheit von unter 5 Mikron vermahlt.

Beispiel D:

Ein emulgierbares Konzentrat wird erhalten aus
15 Gewichtsteilen Wirkstoff
75 Gewichtsteilen Cyclohexanon als Lösungsmittel
und 10 Gewichtsteilen oxäthyliertes Nonylphenol (10 AeO) als Emulgator.

## B. Chemische Beispiele

### Beispiel 1

N-(4-Methylbenzthiazol-2-yl)-2,6-dinitro-3-chlor-4-trifluormethylanilin

Zu einer Lösung von 8,2 g 2-Amino-4-methylbenzthiazol in 150 ml Dimethylformamid fügte man unter Rühren bei 0°C bis -5°C 7 g fein gepulvertes, 88 %iges Kaliumhydroxid. Anschließend tropfte man bei dieser Temperatur 15,2 g 2,4-Dichlor-3,5-dinitro-benzotrifluorid in 50 ml Dimethylformamid ein. Das Reaktionsgemisch wurde zwei Stunden bei 0°C und 12 Stunden bei Raumtemperatur gerührt, in ca. 3 l Wasser eingegossen und mit konz. Salzsäure auf pH 1-2 angesäuert. Man extrahierte dreimal mit Methylenchlorid, wusch die vereinigten Extrakte mit Wasser, trocknete über Natriumsulfat und destillierte das Lösungsmittel im Vakuum ab. Das Reaktionsprodukt fällt in quantitativer Ausbeute in Form hellbrauner Kristalle an.
Fp.: 184°C Z.

Beispiel 2

N-(6-Chlorbenzthiazol-2-yl)-2,6-dinitro-3-chlor-4-trifluormethylanilin

Zu einer Lösung von 9,2 g 2-Amino-6-chlorbenzthiazol in 150 ml Tetrahydrofuran fügte man unter Rühren bei 0°C 7 g fein gepulvertes, 88 %iges Kaliumhydroxid. Anschließend tropfte man bei dieser Temperatur 15,2 g 2,4-Dichlor-3,5-dinitrobenzotrifluorid in 50 ml Tetrahydrofuran ein. Das Reaktionsgemisch wurde zwei Stunden bei 0°C und 10 Stunden bei Raumtemperatur gerührt, in ca. 3 l Wasser eingegossen und mit konz. Salzsäure auf pH 1-2 angesäuert. Man extrahierte dreimal mit Ethylacetat, wusch die vereinigten Extrakte mit Wasser, trocknete über Natriumsulfat und destillierte das Lösungsmittel im Vakuum ab. Das Rohprodukt (22,2 g entspricht 98 % der Theorie) bildet dunkelbraune Kristalle, die durch Chromatographie über Kieselgel gereinigt werden können.

Fp.: 191°C Z  Orangefarbene Kristalle.

Die Verbindungen der folgenden Tabelle 1 lassen sich auf entsprechende Weise herstellen.

Tabelle 1

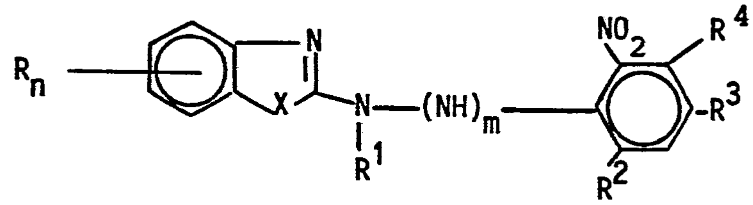

| | $R_n$ | X | $R^1$ | $R^2$ | $R^3$ | $R^4$ | m | Phys.Daten/Fp. |
|---|---|---|---|---|---|---|---|---|
| 3 | 6-Cl | O | $CH_3$ | $NO_2$ | $CF_3$ | H | 0 | 125°C |
| 4 | 6-Cl | O | H | $NO_2$ | $CF_3$ | Cl | 0 | 201°C |
| 5 | 6-Cl | O | $CH_3$ | $NO_2$ | $CF_3$ | Cl | 0 | 120°C |
| 6 | 4-Cl; 7-Cl | S | H | $NO_2$ | $CF_3$ | Cl | 0 | 206°C |
| 7 | 4-Cl; 5-Cl | S | H | $NO_2$ | $CF_3$ | Cl | 0 | 85°C |
| 8 | 4-$CF_3$, 5-Cl | S | H | $NO_2$ | $CF_3$ | Cl | 0 | 180°C |
| 9 | 4-Cl, 7-$CF_3$ | S | H | $NO_2$ | $CF_3$ | Cl | 0 | 171°C |
| 10 | 4-$CF_3$ | S | H | $NO_2$ | $CF_3$ | Cl | 0 | 75°C |
| 11 | 6-F | S | H | $NO_2$ | $CF_3$ | Cl | 0 | 171°C |
| 12 | 4-Cl, 6-Cl | S | H | $NO_2$ | $CF_3$ | Cl | 0 | 188°C |
| 13 | 6-Cl | S | H | $NO_2$ | $CO_2C_2H_5$ | H | 0 | Harz |
| 14 | 6-$OCH_3$ | S | H | $NO_2$ | $CF_3$ | Cl | 0 | 74°C |
| 15 | 6-$OC_2H_5$ | S | H | $NO_2$ | $CF_3$ | Cl | 0 | 115°C |
| 16 | 6-$CH_3$ | S | H | $NO_2$ | $CF_3$ | Cl | 0 | 120°C |
| 17 | 6-Cl | O | H | $NO_2$ | $CF_3$ | H | 1 | 72°C |
| 18 | 6-Cl | O | H | $NO_2$ | $CF_3$ | Cl | 1 | 205°C |
| 19 | 6-Cl | S | H | $NO_2$ | $CF_3$ | H | 1 | 103°C |
| 20 | 6-Cl | S | H | $NO_2$ | $CF_3$ | Cl | 1 | 201°C |
| 21 | 6-Cl | S | $CH_3$ | $NO_2$ | $CF_3$ | Cl | 1 | 79-85°C |
| 22 | 6-Cl | S | $CH_3$ | $NO_2$ | $CF_3$ | H | 1 | 73-77°C |
| 23 | 6-Cl | O | $CH_3$ | $NO_2$ | $CF_3$ | H | 1 | |
| 24 | 6-Cl | O | $CH_3$ | $NO_2$ | $CF_3$ | Cl | 1 | |
| 25 | 6-Cl | S | H | $CF_3$ | $NO_2$ | H | 0 | 90°C |
| 26 | 6-Cl | O | H | $CF_3$ | $NO_2$ | H | 0 | 61°C |
| 27 | 4-Cl, 7-Cl | S | H | $CF_3$ | $NO_2$ | H | 0 | |

Fortsetzung Tabelle 1

| | $R_n$ | X | $R^1$ | $R^2$ | $R^3$ | $R^4$ | m | Phys.Daten /Fp. |
|---|---|---|---|---|---|---|---|---|
| 28 | 4-$CF_3$ | S | H | $NO_2$ | $CF_3$ | H | 0 | 203°C |
| 29 | 6-Cl | S | H | $NO_2$ | $CF_3$ | H | 0 | 97°-102°C |
| 30 | 6-$NO_2$ | S | H | $NO_2$ | $CF_3$ | Cl | 0 | 112°C |
| 31 | 4-$CF_3$, 6-Cl | S | H | $NO_2$ | $CF_3$ | Cl | 0 | |
| 32 | 4-$CH_3$, 6-Cl | S | H | $NO_2$ | $CF_3$ | Cl | 0 | 143°C |
| 33 | 6-Cl | S | H | $NO_2$ | CN | H | 0 | 137°C |
| 34 | 6-Cl | S | H | $NO_2$ | $CCl_3$ | H | 0 | 114°C |
| 35 | 6-$CF_3$ | S | H | $NO_2$ | $CF_3$ | Cl | 0 | 72°C |
| 36 | 4-Cl, 6-$CF_3$ | S | H | $NO_2$ | $CF_3$ | Cl | 0 | |
| 37 | 6-$CF_3$ | O | H | $NO_2$ | $CF_3$ | Cl | 0 | |
| 38 | 4-$CF_3$, 6-Cl | O | H | $NO_2$ | $CF_3$ | Cl | 0 | |
| 39 | 6-$CF_3$ | S | H | $NO_2$ | $CF_3$ | Cl | 1 | |
| 40 | 6-$CF_3$ | O | H | $NO_2$ | $CF_3$ | Cl | 1 | |
| 41 | 6-$CF_3$ | S | H | $NO_2$ | $CF_3$ | H | 0 | |
| 42 | 6-$CF_3$ | O | H | $NO_2$ | $CF_3$ | H | 0 | |
| 43 | 6-Cl | S | $CH_3CO$ | $NO_2$ | $CF_3$ | Cl | 0 | |
| 44 | 6-SCN | S | H | $NO_2$ | $CF_3$ | Cl | 0 | 240°C |
| 45 | 6-SCN | S | H | $NO_2$ | $CF_3$ | H | 0 | 207°C |
| 46 | 6-Cl | O | H | $NO_2$ | $COOC_2H_5$ | Cl | 0 | 171°C |
| 47 | 6-$OCH_3$ | S | H | $CF_3$ | $NO_2$ | H | 0 | 65°C |

## c. Biologische Beispiele

### Beispiel I

Biomalzagar wird mit den beanspruchten Verbindungen (Tabelle I) in Konzentrationen von jeweils 2000 und 500 ppm Wirkstoff versetzt. Nach dem Erstarren des Agars erfolgt die Beimpfung mit verschiedenen Botrytis cinerea-Kulturen, die Benzimidazol- und Iprodion-sensibel bzw. -resistent sind. Jeweils 20 µl einer Sporensuspension werden auf das Zentrum der Agarplatte aufgebracht. Die Versuche werden 6 Tage nach Beimpfung ausgewertet. Der Wirkungsgrad der Wirkstoffe wird ausgedrückt in % im Vergleich zur Kontrolle (Agarmedium ohne Wirkstoff).

Tabelle I

Botrytis cinerea, BCM- und Iprodion-sensibler (s) und -resistenter (r)-Stamm

| Verbindung gemäß Beispiel Nr. | Wirkungsgrad in % bei ppm Wirkstoff | | | |
|---|---|---|---|---|
| | 2000 | | 500 | |
| | s | r | s | r |
| 1 | 100 | 50 | 100 | 50 |
| 15 | 100 | 50 | 80 | 50 |
| 16 | 100 | 50 | 80 | 50 |
| 14 | 100 | 80 | 100 | 50 |
| 7 | 100 | 100 | 100 | 100 |
| 8 | 100 | 100 | 100 | 100 |
| 4 | 100 | 100 | 100 | 100 |
| 6 | 100 | 50 | 100 | 100 |
| 2 | 100 | 100 | 100 | 100 |
| 3 | 100 | 100 | 100 | 100 |
| 5 | 100 | 100 | 100 | 100 |
| Kontrolle | 0 | | | |

./.

Beispiel 2

Biomalzagar wird mit den beanspruchten Verbindungen (TabelleII) in Konzentrationen von jeweils 125, 30 und 8 ppm Wirkstoff versetzt. Nach dem Erstarren des Agars erfolgt die Beimpfung mit Pseudocercosporella-Kulturen. Jeweils 20 µl einer Sporensuspension werden auf das Zentrum der Agarplatte aufgebracht. Die Versuche werden 6 Tage nach Beimpfung ausgewertet. Der Wirkungsgrad der Wirkstoffe wird ausgedrückt in % im Vergleich zur Kontrolle (Agarmedium ohne Wirkstoff).

Tabelle II

| Verbindung gemäß Beispiel Nr. | Wirkungsgrad gegenüber Pseudocercosporella herpotrichoides in % bei ppm Wirkstoff | | | |
|---|---|---|---|---|
| | 250 | 125 | 30 | 8 |
| 1 | 100 | 100 | 100 | 80 |
| 8 | 100 | 100 | 100 | 100 |
| 4 | 100 | 100 | 100 | 100 |
| 6 | 100 | 100 | 100 | 100 |
| 2 | 100 | 100 | 100 | 100 |
| 15 | 100 | 100 | 80 | - |
| 16 | 100 | 100 | - | - |
| 14 | 100 | 100 | 80 | - |
| 13 | 100 | 80 | 50 | - |
| 11 | 100 | 100 | 50 | - |
| Kontrolle | 0 | | | |

./.

Patentansprüche

1. Verbindungen der Formel I,

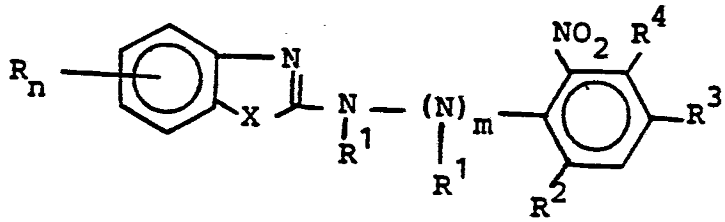

I

worin X = 0 oder S,

R = Halogen, CN, SCN, $NO_2$, $(C_1-C_4)$Alkyl, $(C_1-C_4)$ Alkoxy, $(C_1-C_4)$Alkylthio, wobei die drei letztgenannten Reste ganz oder teilweise halogeniert sein können, oder Phenoxy, das durch Halogen, $(C_1-C_4)$Alkyl, $(C_1-C_4)$Alkoxy, Halo$(C_1-C_4)$alkoxy oder Halo$(C_1-C_4)$alkyl substituiert sein kann,

$R^1$ = jeweils unabhängig voneinander H,$(C_1-C_4)$Alkyl oder $(C_1-C_4)$Alkyl-carbonyl

$R^2$, $R^3$ = jeweils unabhängig voneinander Halogen, $NO_2$, Halo$(C_1-C_4)$alkyl, CN, COOH oder $(C_1-C_4)$-Alkoxy-carbonyl,

$R^4$ = H, Halogen, $(C_1-C_4)$Alkyl oder Halo$(C_1-C_4)$alkyl

m = 0 oder 1 und

n = 0 bis 4

bedeuten, oder deren Salze, mit der Maßgabe, daß nicht beide Reste $R^2$, $R^3$ gleichzeitig im selben Molekül $NO_2$ bedeuten dürfen.

2. Verbindungen der Formel I von Anspruch 1, worin R in Position 4 oder 6 des Benzoxazolyl- bzw. Benzthiazolylrestes steht und F, Cl oder $CF_3$ bedeutet und worin $R^2$= $NO_2$ oder $CF_3$, $R^3$ = $CF_3$, $CCl_3$, CN, COOH, $(C_1-C_4)$-Alkoxycarbonyl, Nitro, $R^4$ = H oder Halogen, m = 0 und n = 1 oder 2 bedeuten.

3. Verfahren zur Herstellung der Verbindungen der Formel I von Anspruch 1, dadurch gekennzeichnet, daß man

a) eine Verbindung der Formel II

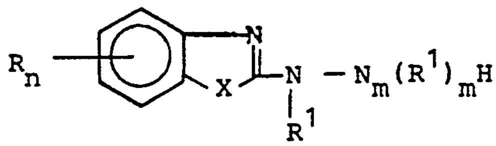 (II)

mit einer Verbindung der Formel III,

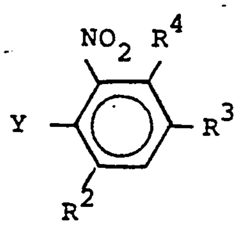 (III)

worin Y Halogen bedeutet, oder

b) eine Verbindung der Formel IV

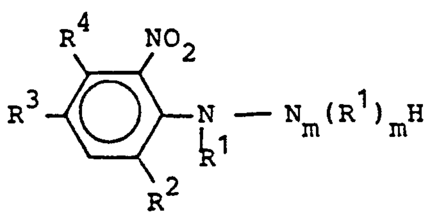 (IV)

mit einer Verbindung der Formel V,

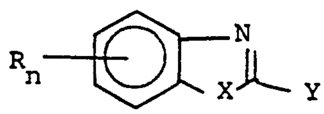 (V)

worin Y die Bedeutung wie in Formel III besitzt, umsetzt.

4. Fungizide Mittel, dadurch gekennzeichnet, daß sie eine wirksame Menge einer Verbindung der Formel I von Ansprüchen 1 oder 2 enthalten

5. Verwendung der Verbindungen der Formel I von Ansprüchen 1 oder 2 zur Bekämpfung von Schadpilzen.

6. Verfahren zur Bekämpfung von Schadpilzen, dadurch gekennzeichnet, daß man auf diese oder die von Ihnen befallenen Pflanzen, Substrate oder Anbauflächen eine wirksame Menge einer Verbindung der Formel I von Ansprüchen 1 oder 2 appliziert.

Patentansprüche Österreich:

1. Verfahren zur Herstellung der Verbindungen der Formel I,

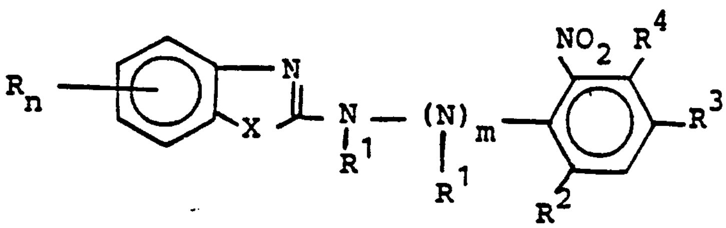

I

worin X = O oder S,

R = Halogen, CN, SCN, $NO_2$, $(C_1-C_4)$Alkyl, $(C_1-C_4)$ Alkoxy, $(C_1-C_4)$Alkylthio, wobei die drei letztgenannten Reste ganz oder teilweise halogeniert sein können, oder Phenoxy, das durch Halogen, $(C_1-C_4)$Alkyl, $(C_1-C_4)$Alkoxy, Halo$(C_1-C_4)$alkoxy oder Halo$(C_1-C_4)$alkyl substituiert sein kann,

$R^1$ = jeweils unabhängig voneinander H, $(C_1-C_4)$Alkyl oder $(C_1-C_4)$Alkyl-carbonyl

$R^2$, $R^3$ = jeweils unabhängig voneinander Halogen, $NO_2$, Halo$(C_1-C_4)$alkyl, CN, COOH oder $(C_1-C_4)$-Alkoxy-carbonyl,

$R^4$ = H, Halogen, $(C_1-C_4)$Alkyl oder Halo$(C_1-C_4)$alkyl

m = 0 oder 1 und

n = 0 bis 4

bedeuten, oder deren Salze, mit der Maßgabe, daß nicht beide Reste $R^2$, $R^3$ gleichzeitig im selben Molekül $NO_2$ bedeuten dürfen, dadurch gekennzeichnet, daß man

a) eine Verbindung der Formel II

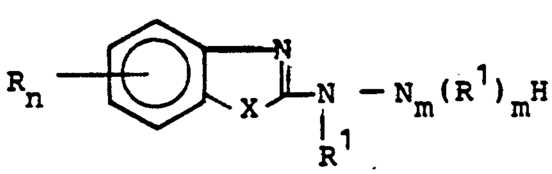

(II)

mit einer Verbindung der Formel III,

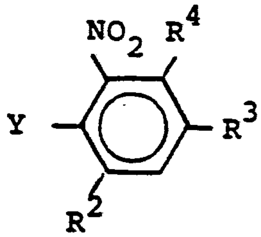

(III)

worin Y Halogen bedeutet, oder

b) eine Verbindung der Formel IV

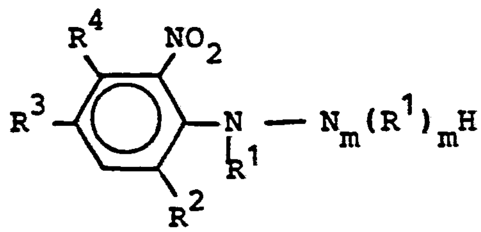

(IV)

mit einer Verbindung der Formel V,

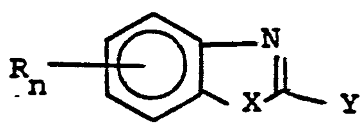

(V)

worin Y die Bedeutung wie in Formel III besitzt, umsetzt.

2. Verfahren gemäß Anspruch 1, wobei in den Formeln I bis V R in Position 4 oder 6 des Benzoxazolyl- bzw. Benzthiazolylrestes steht und F, Cl oder $CF_3$ bedeutet, $R^2$= $NO_2$ oder $CF_3$, $R^3$ = $CF_3$, $CCl_3$, CN, COOH, $(C_1-C_4)$-Alkoxycarbonyl, Nitro, $R^4$= H oder Halogen, m= O und n= 1 oder 2 bedeuten.

3. Fungizide Mittel, dadurch gekennzeichnet, daß sie eine wirksame Menge einer Verbindung der Formel I enthalten

4. Verwendung der Verbindungen der Formel I von Anspruch 1 zur Bekämpfung von Schadpilzen.

5. Verfahren zur Bekämpfung von Schadpilzen, dadurch gekennzeichnet, daß man auf diese oder die von Ihnen befallenen Pflanzen, Substrate oder Anbauflächen eine wirksame Menge einer Verbindung der Formel I appliziert.

HOE 86/F 093

Patentansprüche Spanien:

1. Verfahren zur Herstellung der Verbindungen der Formel I,

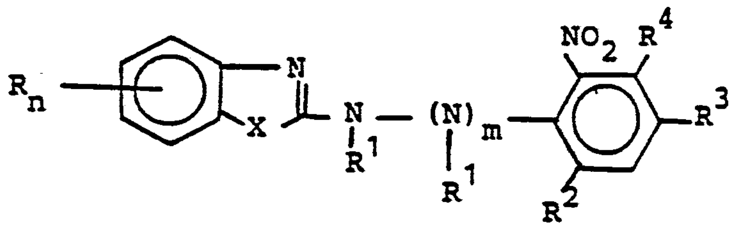

I

worin X = O oder S,

R = Halogen, CN, SCN, $NO_2$, $(C_1-C_4)$Alkyl, $(C_1-C_4)$ Alkoxy, $(C_1-C_4)$Alkylthio, wobei die drei letztgenannten Reste ganz oder teilweise halogeniert sein können, oder Phenoxy, das durch Halogen, $(C_1-C_4)$Alkyl, $(C_1-C_4)$Alkoxy, Halo$(C_1-C_4)$alkoxy oder Halo$(C_1-C_4)$alkyl substituiert sein kann,

$R^1$ = jeweils unabhängig voneinander H,$(C_1-C_4)$Alkyl oder $(C_1-C_4)$Alkyl-carbonyl

$R^2$, $R^3$ = jeweils unabhängig voneinander Halogen, $NO_2$, Halo$(C_1-C_4)$alkyl, CN, COOH oder $(C_1-C_4)$-Alkoxy-carbonyl,

$R^4$ = H, Halogen, $(C_1-C_4)$Alkyl oder Halo$(C_1-C_4)$alkyl

m = 0 oder 1 und

n = 0 bis 4

bedeuten, oder deren Salze, mit der Maßgabe, daß nicht beide Reste $R^2$, $R^3$ gleichzeitig im selben Molekül $NO_2$ bedeuten dürfen, dadurch gekennzeichnet, daß man

a) eine Verbindung der Formel II

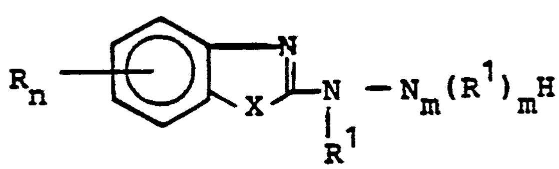

(II)

mit einer Verbindung der Formel III,

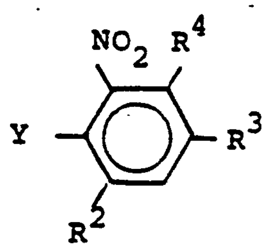

(III)

worin Y Halogen bedeutet, oder

b) eine Verbindung der Formel IV

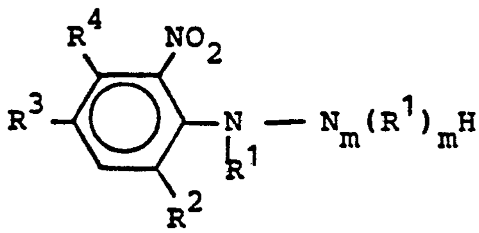

(IV)

mit einer Verbindung der Formel V,

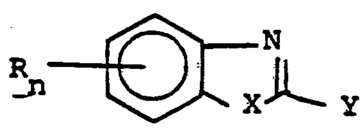

(V)

worin Y die Bedeutung wie in Formel III besitzt, umsetzt.

2. Verfahren gemäß Anspruch 1, wobei in den Formeln I bis V R in Position 4 oder 6 des Benzoxazolyl- bzw. Benzthiazolylrestes steht und F, Cl oder $CF_3$ bedeutet, $R^2$= $NO_2$ oder $CF_3$, $R^3$ = $CF_3$, $CCl_3$, CN, COOH, $(C_1-C_4)$-Alkoxycarbonyl, Nitro, $R^4$= H oder Halogen, m= O und n= 1 oder 2 bedeuten.

3. Verwendung der Verbindungen der Formel I von Anspruch 1 zur Bekämpfung von Schadpilzen.

4. Verfahren zur Bekämpfung von Schadpilzen, dadurch gekennzeichnet, daß man auf diese oder die von Ihnen befallenen Pflanzen, Substrate oder Anbauflächen eine wirksame Menge einer Verbindung der Formel I appliziert.